# EUROPÄISCHE PATENTSCHRIFT

(11) **EP 3 721 236 B1**
(45) Veröffentlichungstag und Bekanntmachung des Hinweises auf die Patenterteilung: **02.06.2021**
(21) Anmeldenummer: 19700220.7
(22) Anmeldetag: 08.01.2019
(51) Int. Cl.: G01N 33/94, C12Q 1/34, C07C 213/02, C07C 249/02

(54) **QUANTITATIVE ACETAMINOPHEN-ANALYTIK**
QUANTITATIVE ACETAMINOPHEN ANALYTICS
ANALYSE QUANTITATIVE D'ACÉTAMINOPHÈNE

(30) Priorität: 10.01.2018 DE 102018100426
(43) Veröffentlichungstag der Anmeldung: 14.10.2020
(73) Patentinhaber: DiaSys Diagnostic Systems GmbH, 65558 Holzheim (DE)
(72) Erfinder: GRIMMLER, Matthias, 65604 Elz (DE); THÖNGES, Detlef, 65550 Limburg (DE); SCHU, Pia, 66839 Schmelz (DE); MENZENBACH, Carolin, 56593 Pleckhausen (DE); KRECKEL, Laura, 65558 Gückingen (DE)
(74) Vertreter: WSL Patentanwälte Partnerschaft mbB
(86) Internationale Anmeldenummer: PCT/EP2019/050291
(87) Internationale Veröffentlichungsnummer: WO 2019/137890

(56) Entgegenhaltungen:
- EP-A2- 0 750 197
- JALIL TAVAKOLI AFSHARI ET AL: "Rapid spectrophotometric method for the quantitation of acetaminophen in serum", ANALYTICA CHIMICA ACTA, ELSEVIER, AMSTERDAM, NL, Bd. 443, 1. Januar 2001 (2001-01-01), Seiten 165-169, XP008146313, ISSN: 0003-2670
- MORRIS H C ET AL: "Development and validation of an automated enzyme assay for paracetamol (acetaminophen)", CLINICA CHIMICA ACTA, ELSEVIER BV, AMSTERDAM, NL, Bd. 187, Nr. 2, 28. Februar 1990 (1990-02-28), Seiten 95-104, XP023398129, ISSN: 0009-8981, DOI: 10.1016/0009-8981(90)90335-P [gefunden am 1990-02-28]
- HAMMOND P M ET AL: "Development of an enzyme-based assay for acetaminophen", ANALYTICAL BIOCHEMISTRY, ELSEVIER, AMSTERDAM, NL, Bd. 143, Nr. 1, 15. November 1984 (1984-11-15), Seiten 152-157, XP024820133, ISSN: 0003-2697, DOI: 10.1016/0003-2697(84)90570-0 [gefunden am 1984-11-15]

## Beschreibung

### GEGENSTAND DER ERFINDUNG

Die Erfindung betrifft ein Verfahren zur quantitativen Bestimmung der Menge an Acetaminophen in einer Probe und einen Reagenziensatz zur Verwendung in dem erfindungsgemäßen Verfahren.

### HINTERGRUND DER ERFINDUNG

Acetaminophen ist die in den USA geläufige Bezeichnung für den v.a. in Europa unter dem Namen Paracetamol bekannten Wirkstoff *N*-(4-Hydroxyphenyl)acetamid) mit der Formel (I)

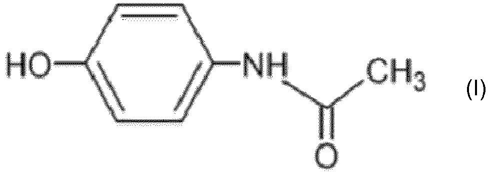

Acetaminophen ist ein weit verbreiteter Wirkstoff, der aufgrund seiner analgetischen und antipyretischen Eigenschaften in vielen frei verkäuflichen und rezeptpflichtigen Präparaten Verwendung findet. Bei überdosierter Einnahme kann Acetaminophen schwere Leber- und Nierenschädigungen oder sogar den Tod verursachen.

Nach akuter Acetaminophen-Überdosis zeigt der Patient im Frühstadium häufig nur wenige oder gar keine Symptome. Der einzige verlässliche Frühindikator für eine Diagnose ist die quantitative Messung der Acetaminophen-Konzentration im Blut, Blutserum oder Blutplasma. Der klinische Nachweis einer Leber- und Nierenschädigung kann hingegen in der Regel frühestens 24 Stunden nach Einnahme erbracht werden, d. h. deutlich nach dem Zeitpunkt, an dem das prophylaktische Gegenmittel N-Acetylcystein wirksam verabreicht werden kann.

Vorfälle von sowohl versehentlicher als auch absichtlicher Überdosierung haben stark zugenommen, und die Diagnose und Behandlung solcher Überdosierungen verlangt eine frühe und zuverlässige Messung des Gehaltes an Acetaminophen im Blut des Patienten.

### STAND DER TECHNIK

Die in der Praxis wohl am weitesten verbreiteten diagnostischen Tests zur Bestimmung des Gehaltes an Acetaminophen basieren auf der hydrolytischen Spaltung von Acetaminophen, wobei das Spaltprodukt p-Aminophenol anschließend unter oxidativen Bedingungen entweder mit einem Kresol (Methylphenol) oder einem Xylenol (Dimethylphenol) zu einem farbigen Indophenol umgesetzt wird. Beispielsweise offenbaren US 8,715,952 B2 sowie J T Afshari et al: "Rapid spectrophotometric method for the quantitation of acetaminophen in serum", ANAL CHIM ACTA, 443, 2001, 165-169 Verfahren, bei denen durch oxidative Kupplung von hydrolysiertem Acetaminophen mit einem Xylenol ein Indolfarbstoff entsteht, dessen Quantität dann photospektrometrisch bestimmt wird. H C Morris et al: "Development and validation of an automated enzyme assay for paracetamol (acetaminophen)", CLINICA CHIMICA ACTA, 187(2), 1990, 95-104 und P M Hammond et al: "Development of an enzyme-based assay for acetaminophen", ANAL BIOCHEM, 143(1), 1984, 152-157 offenbaren weitere Verfahren zur quantiativen Bestimmung von Acetaminophen unter Verwendung eines Hydroxyquinolins bzw. eines Tetrazolium-Farbstoffs.

Die in den genannten Tests eingesetzten Phenole sind toxisch und ihre Stabilität ist teilweise begrenzt, was die Messzuverlässigkeit beeinträchtigen kann. Jedenfalls ist die erforderliche Messgenauigkeit in manchen Fällen nicht über den gesamten diagnostisch relevanten Bereich mit hinreichender Zuverlässigkeit reproduzierbar.

Alternative Methoden zur zuverlässigen Bestimmung des Gehaltes an Acetaminophen sind beispielsweise chromatographische Verfahren. Sowohl die Gas-Flüssigkeitschromatographie als auch die High-Performance-Flüssigkeitschromatographie (HPLC) stellen zwar verlässliche und genaue Methoden zur Bestimmung dar, erfordern jedoch einen entsprechend hohen Arbeitsaufwand und kostenintensive Chromatographieapparate.

Obwohl sie meist stärkere Interferenzen mit biologischen Molekülen zeigen, sind Enzym-basierte Assays aufgrund ihrer Einfachheit und Ökonomie bevorzugt. Nichtsdestotrotz führen Wechselwirkungen sowie spektrale und chemische Interferenzen mit anderen Biomolekülen und anderen therapeutischen Reagenzien nicht selten zu Fehlindikationen.

In EP 0 750 197 A2 werden alternative Kopplungsmittel mit einem Chinolingrundgerüst beschrieben, die jedenfalls mit in der zu untersuchenden Probe enthaltenem Billirubin nicht interferieren, da sie nach deren Reaktion mit dem Acetaminophen-Hydrolyseprodukt p-Aminophenol zu Indolfarbstoffen führen, deren Absorbtionsmaximum mit einem Wert von mehr als 0.3 bei einer Wellenlänge von etwa 670 nm liegt. Für diese alternativen Kopplungsmittel wird außerdem beschrieben, dass sie aufgrund ihrer guten Wasserlöslichkeit schneller mit p-Aminophenol reagieren, um dabei den Indolfarbstoff auszubilden.

Diese Kopplungsmittel adressieren jedoch nicht das Problem der Wechselwirkungen mit N-Acetylcystein (NAC), das insbesondere dann in einer zu untersuchenden Probe enthalten sein kann, wenn es bei einem bereits diagnostizierten Acetaminophen-Intox als Antidot gegeben worden ist. Außerdem sind bei Verwendung dieser Verbindungen auch Wechselwirkungen mit Hämoglobin und Hämolysat nicht ausgeschlossen. Des weiteren erfordern die Systeme, die mit den aus dem Stand der Technik bekannten Kopplungsmitteln arbeiten, relativ große Probenvolumina, die jedoch insbesondere bei

Patientenproben der Pädiatrie nicht immer verfügbar sind, da hier gerade im Zustand des akuten Acetaminophen-Intox nur sehr geringe Blutmengen abgenommen werden können.

### AUFGABE

Nach alledem besteht ein Bedarf nach einem verbesserten Verfahren zur quantitativen Bestimmung der Menge an Acetaminophen in einer Probe mit noch höherer Präzision, einer noch höheren Sensitivität und noch weniger Wechselwirkungen und weniger spektralen und chemischen Interferenzen mit anderen in der Probe enthaltenen Verbindungen, und es war daher die Aufgabe der vorliegenden Erfindung eine entsprechendes Verfahren bereitzustellen.

### BESCHREIBUNG DER ERFINDUNG

Diese Aufgabe wird erfindungsgemäß gelöst durch ein Verfahren der eingangs beschriebenen Art, bei dem man ein Reaktionsgemisch erzeugt, indem man Acetaminophen mit der Formel (I) 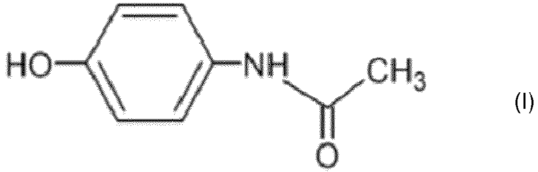 hydrolysiert und das daraus hervorgehende p-Aminophenol mit der Formel (II) 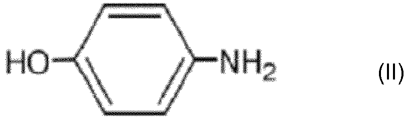 mit einer Verbindung der allgemeinen Formel (III) 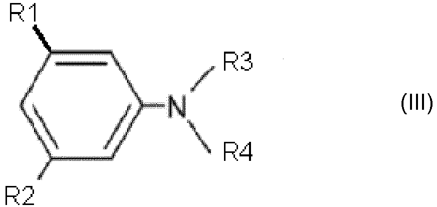 in Gegenwart eines Oxidationsmittels zu einer Verbindung der allgemeinen Formel (IV) umsetzt 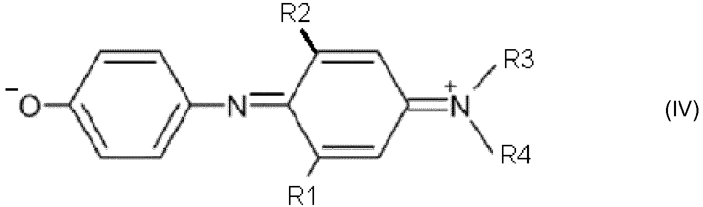 wobei in den Formeln (III) und (IV)
- R1 und R2 unabhängig voneinander ausgewählt sind unter H, -CH₃, und -OCH₃,
- R3 -C₂H₅ ist und
- R4 ein C₁₋₄-Alkylrest mit einer endständigen Sulfonatgruppe ist,
mit der Maßgabe, dass wenigstens einer von R1 und R2 -OCH₃ ist und/oder R4 zusätzlich wenigstens einen OH-Substituenten aufweist,
und anschließend die Menge der Verbindung der allgemeinen Formel (IV) in dem Reaktionsgemisch photometrisch bestimmt.

Mit dem erfindungsgemäßen Verfahren kann die in einer zu untersuchenden Probe enthaltene Menge an Acetaminophen quantitativ ermittelt werden. Bei der zu untersuchenden Probe kann es sich entweder um eine aus einem Lebewesen entnommene Probe handeln oder um eine Probe, die aus sonstigen natürlichen Quellen (z.B. Mikroorganismen) oder künstlichen Quellen (z.B. Zwischen- und Endprodukte eines technischen Herstellungsprozesses) stammt.

In vielen Anwendungsfällen handelt es sich bei der zu untersuchenden Probe um eine flüssige Probe, d.h. um eine Flüssigkeit, in der sich eine zu bestimmende Menge an Acetaminophen befindet oder zumindest vermutet wird, und typische Beispiele für zu untersuchende flüssige Proben sind aus dem Körper eines Lebewesens isolierte Körperflüssigkeiten, wie z.B. Blut, Speichel oder Urin.

In den Fällen, in denen es sich bei der Probe ursprünglich um einen Feststoff handelt, muss für Zwecke der Durchführung des erfindungsgemäßen Verfahrens zunächst das darin enthaltene Acetaminophen quantitativ in einer Flüssigkeit gelöst werden. Auch zu untersuchende Proben, die flüssig sind, können bei bestimmten Ausführungsformen vor der Ausführung des erfindungsgemäßen Verfahrens vorbehandelt werden, z.B. durch Aufreinigung, durch Abtrennung einzelner Komponenten oder durch Aufkonzentrierung. Bei alternativen Ausführungsformen der Erfindung wird die zu untersuchende Probe dagegen ohne Vorbehandlung nach dem erfindungsgemäßen Verfahrens analysiert, d.h. ohne vorherige Aufreinigung, Abtrennung einzelner Komponenten oder Aufkonzentrierung.

Bei dem erfindungsgemäßen Verfahren wird Acetaminophen hydrolysiert. Dabei findet an der N-Acylgruppe des Acetaminophens eine Hydrolysereaktion statt, deren Produkte der abgespaltene Acylrest (hier Essigsäure) und p-Aminophenol sind. Bei der Hydrolysereaktion kann es sich um eine saure, alkalische oder enzymatische Hydrolysereaktion handeln. Besonders bevorzugt läuft diese Reaktion enzymatisch beispielsweise unter Einsatz einer Arylacylamidase ab.

Die Hydrolyse kann innerhalb weniger Stunden erfolgen, bevorzugt innerhalb von weniger als 2 Stunden. Besonders bevorzugt läuft die Hydrolyse innerhalb weniger Minuten oder Sekunden ab. Hierzu kann das Reaktionsgemisch eine Amidohydrolase enthalten und/oder entsprechend temperiert werden und/oder ein für die Hydrolyse bevorzugter pH-Wert eingestellt werden.

Ein für die Hydrolyse bevorzugter pH-Wert ist im stark sauren Bereich, d.h. bei einem pH-Wert von <2, oder im stark alkalischen Bereich, d.h. bei einem pH-Wert von >10,5. Besonders bevorzugt ist ein pH Wert über 10,6.

Um die Konzentration und damit die Menge an Acetaminophen in der Probe quantitativ zu bestimmen, wird das in der Probe enthaltene Acetaminophen erfindungsgemäß auch quantitativ zu p-Aminophenol umgesetzt und dessen Konzentration durch quantitative Umsetzung mit der Verbindung der allgemeinen Formel (III) zu einer Verbindung der allgemeinen Formel (IV) anhand der anschließenden photometrischen Konzentrationsbestimmung der Verbindung der allgemeinen Formel (IV) bestimmt.

Quantitativ bezeichnet in diesem Zusammenhang eine Ausbeute der jeweiligen Reaktion von >99%, beispielsweise bei der Hydrolyse eine Ausbeute an p-Aminophenol >99%.

Das Reaktionsgemisch enthält erfindungsgemäß Verbindungen der allgemeinen Formel (III), die eine negativ geladene Sulfonatgruppe enthalten. Es hat sich als besonders vorteilhaft herausgestellt, dass gerade diese Verbindungen eine sehr gute Löslichkeit in polaren Lösemitteln wie beispielsweise Wasser und polaren Lösemittelgemischen aufweisen. Die Gegenionen der negativ geladenen Seitengruppen können Alkali- oder Erdalkalimetalle oder organische Kationen sein. Sind die Gegenionen Alkali oder Erdalkalimetalle so sind Natrium, Kalium und Calcium bevorzugt. Sind die Gegenionen organische Kationen so sind Ammoniumionen und Phosphoniumionen bevorzugt.

Bei bestimmten Ausführungsformen sind die Verbindungen ausgewählt aus der Gruppe bestehend aus N-Ethyl-N-(3-sulfopropyl)-m-anisidin (Natriumsalz) **ADPS,** N-Ethyl-N-(2-hydroxy-3-sulfopropyl)-3,5-dimethoxyanilin (Natriumsalz) **DAOS,** N-Ethyl-N-(2-hydroxy-3-sulfopropyl)-3,5-dimethylanilin (Natriumsalz-Monohydrat) **MAOS** und N-Ethyl-N-(2-hydroxy-3-sulfopropyl)-3-methylanilin (Natriumsalz-Dihydrat) **TOOS.** Besonders bevorzugt sind N-Ethyl-N-(3-sulfopropyl)-m-anisidin (Natriumsalz) **ADPS** und N-Ethyl-N-(2-hydroxy-3-sulfopropyl)-3,5-dimethoxyanilin (Natriumsalz) **DAOS.**

Die Verbindungen der allgemeinen Formel (III) bieten den Vorteil gegenüber den im Stand der Technik zur Reaktion mit Acetaminophen genutzten Xylenolfarbstoffen, dass diese eine weitaus geringere Toxizität aufweisen und der Absorptionsbereich hin zur längerwelligen Strahlung verschoben ist. Daher kommt es zu weniger Strahlungsinterferenzen mit Biomolekülen wie Bilirubin, deren Absorptionsmaxima bei kürzeren Wellenlängen liegen. Zudem ist die Neigung der Anilinderivate zur Komplexbildung mit Metallionen im Vergleich zu Xylenolderivaten stark verringert, wodurch eine etwaige störende Wechselwirkung viel geringer ausfällt.

Die Verbindungen der allgemeinen Formel (III) ermöglichen eine Analytik mit extrem geringen Probenvolumina, was insbesondere bei Kleinkinder- und Kinderpatienten entscheidend sein kann, da hier gerade im Zustand des akuten Acetaminophen-Intox nur sehr geringe Blutmengen abgenommen werden können. Während die im Stand der Technik beschriebenen Testsysteme ein Probevolumen von mindestens 6 µL an abgenommenem Blut benötigen, genügen bei den erfindungsgemäßen Testsystemen durch die Anwendung der Verbindungen der allgemeinen Formel (III) als Kopplungsmittel gerade einmal 2 µL.

Ohne dass die vorliegende Erfindung durch diese Theorie in irgendeiner Weise eingeschränkt werden soll, dürfte einer der Gründe für die Möglichkeit der Herabsetzung des benötigten Probevolumens der Umstand sein, dass die Erfinder für die Verbindungen der allgemeinen Formel (III) bis zu 15fach höhere Absorptionsmaxima nachgewiesen werden konnten, wodurch eine wesentlich höhere Sensitivität erreicht wird als dies mit den aus dem Stand der Technik bekannten Systemen möglich ist.

Bestimmte Ausführungsformen des erfindungsgemäßen Verfahrens sind daher insbesondere dadurch gekennzeichnet, dass hierbei das Volumen einer flüssigen Probe, wie z.B. einer nicht vorbehandelten Blutprobe, in der die Menge an Acetaminophen quantitativ bestimmt werden soll, 5 µL oder weniger, vorzugsweise 4 µL oder weniger und noch bevorzugter 3 µL oder weniger betragen kann.

Das Reaktionsgemisch enthält bei bevorzugten Ausführungsformen ein Oxidationsmittel ausgewählt aus der Gruppe bestehend aus Wasserstoffperoxid, Kupfer (II) Salzen, Eisen (III) Salzen wie Ferrocyaniden, Periodatsalzen, Periodatkomplexen, Hypochloriten, Perboraten, Percarbonaten, tert-Butylhydroxyperoxid, Dess-Martin-Periodinan, Harnstoff-Wasserstoffperoxid-Addukt, 1-Hydroxy-1,2-benziodoxol-3(1H)-on-1-oxid (IBX), lod, lodosylbenzol, Kaliumperoxymonosulfat, Mangandioxid, Mangan (II) Salzen und Komplexen, Mangan (III) Salzen und Komplexen, N-Methylmorpholin-N-Oxid, 2-Methylprop-2-yl-hydroperoxid, Tetramethylpiperidin-N-Oxid (TEMPO), Tetrabutylammonium Peroxydisulfat. Bevorzugt sind Periodatsalze, Periodatkomplexe und Wasserstoffperoxid, besonders bevorzugt Periodatsalze und Periodatkomplexe.

Milde Oxidationsmittel wie Mangan (II)- oder Mangan (III) Salze bieten den Vorteil, dass nur ein geringes Ausmaß an Nebenreaktionen mit anderen Substanzen im Reaktionsgemisch auftritt. Stärkere Oxidationsmittel wie Periodate führen hingegen zu einer schnelleren Umsetzung. Wasserstoffperoxid ist aufgrund seiner sehr guten Wasserlöslichkeit vorteilhaft. Wenn Wasserstoffperoxid das Oxidationsmittel in dem Reaktionsgemisch des erfindungsgemäßen Verfahrens ist, kann die Probe zusätzlich das Enzym Peroxidase enthalten, das die Umsetzung katalysiert. Katalysiert bedeutet in diesem Zusammenhang, dass die Aktivierungsenergie der Reaktion herabgesetzt wird und die Umsetzung zeitlich beschleunigt wird.

Die Menge der gebildeten Verbindung der Formel (IV) wird über die Konzentration in dem Reaktionsgemisch bei bekanntem Volumen bestimmt. Die Bestimmung der Konzentration erfolgt photometrisch durch Messung der Absorption oder Transmission bei einer oder mehrerer Frequenzen in einem Wellenlängenbereich der Strahlung von 400 nm bis 800 nm, vorzugsweise 500 nm bis 800 nm, besonders bevorzugt 650 nm bis 800 nm. Ein wesentlicher Vorteil dieses Verfahrens ist, dass der Wellenlängenbereich, in dem die zu untersuchende Frequenz gemessen wird, über 500 nm, besonders bevorzugt über 650 nm liegt und es hierdurch zu geringen spektralen Interferenzen mit anderen absorbierenden Biomolekülen der zu untersuchenden Probe wie beispielsweise konjugiertem und unkonjugiertem Bilirubin, Hämoglobin oder mit Produkten der Hämolyse kommt.

Das Reaktionsgemisch kann erfindungsgemäß weiterhin N-Ethylmaleinimid (NEM) und/oder Maleinimid enthalten. Diese können jedenfalls in einem pH-Bereich von 6,5-7,5 mit Biomolekülen und anderen therapeutischen Reagenzien mit freier Thiolgruppe reagieren, die zu Wechselwirkungen sowie zu spektralen und chemischen Interferenzen bei der Bestimmung der Konzentration der Verbindung der Formel (III) führen können. Überraschenderweise konnten die Erfinder der vorliegenden Anmeldung allerdings feststellen, dass dieser Effekt bei dem erfindungsgemäßen Verfahren auch bei einem pH-Wert von >10 erreicht werden kann, und dies obwohl im erfindungsgemäßen Testsystem gleichzeitig auch noch ein starkes Oxidationsmittel vorliegt, mit dem NEM und/oder Maleinimid normalerweise reagieren müssten.

Das Reaktionsgemisch kann erfindungsgemäß weiterhin ein Komplexierungsreagenz ausgewählt aus der Gruppe bestehend aus Ethylendiaminotetraessigsäure **(EDTA),** Dimercaptopropansulfonsäure, 1,4,7,10-Tetraazacyclododecan-1,4,7,10-tetraessigsäure **(DOTA),** Iminodisuccinat-Tetranatriumsalz, Oxalsäure, Bernsteinsäure, Hydroxysäuren ausgewählt aus der Gruppe bestehend aus Milchsäure, Äpfelsäure, Weinsäure, Citronensäure sowie deren Salzen, Polyphosphaten (ATP und Pyrophosphat), Aminosäuren, Peptiden, Proteinen und Porphyrinen. Bevorzugt sind EDTA, DOTA und Oxalsäure, besonders bevorzugt EDTA. Das Komplexierungsreagenz kann freie oder frei werdende Metallionen binden und dadurch eine Wechselwirkung der Analyten mit diesen, die zu Interferenzen und Ungenauigkeiten bei der Messung führen können, verhindern. Zudem bindet das Komplexierungsreagenz Schwermetallionen, die die Zersetzung des Oxidationsmittels katalysieren und verhindert so eine erleichterte Zersetzung des Oxidationsmittels.

Das Reaktionsgemisch kann erfindungsgemäß weiterhin Enzyme enthalten ausgewählt aus der Gruppe der Amidohydrolasen. Dies sind Biomoleküle, in deren Gegenwart eine biochemische Reaktion schneller abläuft und eine geringere Aktivierungsenergie für die Hydrolysereaktion notwendig ist. Für das Reaktionsgemisch des erfindungsgemäßen Verfahrens sind als Amidohydrolasen besonders Arylacylamidasen (EC-Nr. 3.5.1.13) bevorzugt.

Das Reaktionsgemisch kann erfindungsgemäß weiterhin einen pH-Wert im Bereich von 6,0 bis 12,0, bevorzugt 7,0 bis 11,0, besonders bevorzugt 8,0 bis 10,5 aufweisen. Zur Einstellung dieses pH-Wertes können Puffer ausgewählt aus der Gruppe bestehend aus Phosphat-, Kohlensäure-Carbonat-, Veronal-Acetat-, Ammoniak-Ammonium-, HEPES (4-(2-Hydroxyethyl)-1-piperazinethanesulfonsäure)-, Phosphat-Salz-(PBS), MES (2-(N-Morpholino)-ethansulfonsäure)puffer benutzt werden.

Besonders bevorzugt sind aufgrund ihrer exzellenten Verfügbarkeit und ihrer Fähigkeit freie Metallionen zu binden Phosphat- und Phosphat-Salzpuffer. Dadurch kann bei Verwendung solcher Phosphatpuffer auf zusätzliche Komplexierungsreagenzien verzichtet werden.

Bei dem erfindungsgemäßen Verfahren können weiterhin Substanzen mit konservierender, antimikrobieller, antimykotischer und antibiotischer Wirkung eingesetzt werden. Borsäure und/oder Natriumbenzoat können zur Konservierung der Probe oder des Reaktionsgemisches zugesetzt werden. Die milde antibiotische und desinfizierende Wirkung dieser Substanzen ermöglicht eine langfristige Stabilität der Probe oder des Reaktionsgemisches. Langfristig bedeutet in diesem Zusammenhang mehrere Jahre, jedoch mindestens ein Jahr.

Da nach Inkrafttreten der GHS-Verordnung 1272/2008/EG und der REACH-Änderungs-VO 790/2009/EG Borsäure als reproduktionstoxisch gekennzeichnet ist, ist in diesem Zusammenhang besonders vorteilhaft, dass das Reaktionsgemisch des erfindungsgemäßen Verfahrens nur sehr geringe Mengen Borsäure (Konzentration <0,1%) enthalten muss. Als antimikrobielle Substanz wird bevorzugt ein wasserlösliches Aminoglykosid-Antibiotikum eingesetzt. Beispiele hierfür sind Gentamicin, Amikacin, Apramycin, Geneticin, Kanamycin, Netilmicin, Neomycin, Paromomycin, Spectinomycin, Streptomycin, Tobramycin sowie deren Salze. Weiterhin kann das Reaktionsgemisch des erfindungsgemäßen Verfahrens eine antimykotische Verbindung enthalten. Bevorzugt ist hier Pyridin-2-thiol-1-oxid und dessen Salze, besonders bevorzugt Natriumomadine (Pyrithion).

Zur Ausführung des erfindungsgemäßen Verfahrens kann ein Reagenziensatz verwendet werden. Dementsprechend umfasst die vorliegende Erfindung auch die zur Verwendung eines entsprechenden Reagenziensatzes zur quantitativen Bestimmung von Acetaminophen in einer Probe. Dieser Reagenziensatz kann aus einem oder mehreren flüssigen oder festen Reagenzien oder Reagenzienmischungen bestehen. Die einzelnen Reagenzien oder Reagenzienmischungen können im Verlaufe des Verfahrens in unterschiedlicher Reihenfolge zueinander gegeben werden.

Vorteilhaft ist, wenn ein Reagenz oder eine Reagenzienmischung ein oder mehrere Enzyme umfasst, die in der Reaktion eingesetzt werden. Für dieses Reagenz oder diese Reagenzienmischung ist es vorteilhaft, wenn in diesem oder dieser kein Oxidationsmittel enthalten ist, da dies zu einer negativen Beeinflussung der Enzyme führen kann. Weiterhin ist für dieses Reagenz oder diese Reagenzienmischung vorteilhaft, wenn dieses oder diese einen pH-Puffer enthält, der den pH-Wert im physiologischen Bereich (7.0-7.5) hält und somit die uneingeschränkte Aktivität der Enzyme ermöglicht.

Weiterhin vorteilhaft ist für dieses Reagenz oder diese Reagenzienmischung, wenn dieses oder diese Substanzen mit konservierender, antimikrobieller, antimykotischer und antibiotischer Wirkung, wie im vorherigen Abschnitt beschrieben, enthält. Das Oxidationsmittel kann in einem getrennten Reagenz oder einer Reagenzienmischung mit den Verbindungen der Formeln (I) und (III) vorliegen. Vorteilhaft ist jedoch, wenn das Oxidationsmittel in einem getrennten Reagenz oder einer Reagenzienmischung vorhanden ist, um eine Wechselwirkung mit den Verbindungen der Formel (I) und/oder (III) und/oder der Enzyme zu vermeiden.

Vorteilhaft ist, wenn das Reagenz oder die Reagenzienmischung, das bzw. die das Oxidationsmittel enthält, zusätzlich eine stabilisierende Substanz aufweist, die die Zersetzung des Oxidationsmittel über die Zeit möglichst gering hält. Hierzu können Radikalfänger und Komplexierungsreagenzien zählen. Radikalfänger können ausgewählt sein aus der Gruppe bestehend aus aromatischen Alkoholen wie 2,6-Di-*tert*-butyl-*p*-kresol, verzweigten aliphatischen Alkoholen wie Isopropanol oder *tert*-butanol, Vitamin E und K. Diese stabilisieren freie Radikale, die im Zersetzungsprozess des Oxidationsmittels entstehen und verhindern dadurch die weitere Zersetzung.

Komplexierungsreagenzien sind ausgewählt aus der Gruppe bestehend aus Ethylendiaminotetraessigsäure **(EDTA),** Dimercaptopropansulfonsäure, 1,4,7,10-Tetraazacyclododecan-1,4,7,10-tetraessigsäure **(DOTA),** Iminodisuccinat-Tetranatriumsalz, Oxalsäure, Bernsteinsäure, Hydroxysäuren ausgewählt aus der Gruppe bestehend aus Milchsäure, Äpfelsäure, Weinsäure, Citronensäure sowie deren Salzen, Polyphosphaten (ATP und Pyrophosphat), Aminosäuren, Peptiden, Proteinen und Porphyrinen. Bevorzugt sind EDTA, DOTA und Oxalsäure, besonders bevorzugt EDTA. Die Komplexierungsreagenzien binden Schwermetallionen, die die Zersetzung des Oxidationsmittels katalysieren und verhindern so eine erleichterte Zersetzung des Oxidationsmittels.

Das erfindungsgemäße Verfahren kann zur Bestimmung der Acetaminophen-Konzentration im Blutserum, Blutplasma oder im Blut selbst genutzt werden. Vorteilhaft ist die Bestimmung im Blutserum oder Blutplasma, weil dort die Konzentration an Biomolekülen und Substanzen, die zur Interferenz mit den zu bestimmenden Analyten führen, erheblich verringert ist. Ein Vorteil des erfindungsgemäßen Verfahrens ist jedoch seine extrem hohe Präzision und geringe Störanfälligkeit, wodurch selbst in unaufbereiteten Blutproben der quantitative Nachweis von Acetaminophen durchgeführt werden kann.

Auch solche Proben, bei denen durch Fehler bei der Blutabnahme hämolytische Prozesse in Gang gesetzt wurden, können mit dem erfindungsgemäßen Verfahren zuverlässiger analysiert werden, als dies mit herkömmlichen Testsystemen möglich ist, da das erfindungsgemäße Verfahren gegenüber den hierdurch vermehrt auftretenden Interferenzen weniger störanfällig ist.

Bei manchen Ausführungsformen der Erfindung werden die verschiedenen für die Reaktion eingesetzten Reagenzien in Form eines Reagenziensatzes bereitgestellt, der aus verschiedenen Lösungen besteht, die in separaten Gebinden vorgehalten werden. Beispielsweise kann der Reagenziensatz aus zwei Lösungen Reagenz 1 und Reagenz 2 bestehen, die in separaten Gebinden vorgehalten werden, wobei Reagenz 1 eine erste Reagenzienlösung, die wenigstens eines der benötigten Reagenzien enthält, und Reagenz 2 eine zweite Reagenzienlösung bezeichnet, die wenigstens eine weiteres der benötigten Reagenzien enthält.

Bei bestimmten Ausführungsformen enthält Reagenz 1 in wässriger Lösung beispielsweise
- Periodat und
- N-Ethylmaleinimid.

Bei bestimmten Ausführungsformen liegt der pH-Wert der Lösung von Reagenz 2 im Bereich von 10 bis 11 auf.

Bei diesen Ausführungsformen kann die zweite Reagenzienlösung, Reagenz 2, dann beispielsweise in wässriger Lösung
- Arylacylamidase
- das Kopplungsmittel der allgemeinen Formel (III)
und wahlweise zusätzlich noch Natriumbenzoat und/oder EDTA enthalten.

Bei bestimmten Ausführungsformen liegt der pH-Wert der zweiten Reagenzienlösung R2 im Bereich von pH 6 bis 8, vorzugsweise bei pH 7.

Bei bevorzugten Ausführungsformen sind die erste und/oder die zweite Reagenzienlösung phosphatgepuffert.

Ein Reagenziensatz der vorgenannten Art kann in einem Volumenverhältnis von 1 Teil Reagenz 1 + 1 Teil Reagenz 2, 2 Teile Reagenz 1 + 1 Teil Reagenz 2, 3 Teile Reagenz 1 + 1 Teil Reagenz 2 oder 4 Teile Reagenz 1 + 1 Teil Reagenz 2 vorliegen. Bevorzugt sind die Volumenverhältnisse (Reagenz 1 / Reagenz 2) 1+1 und 2+1, besonders bevorzugt 2+1. Durch die beschriebenen Formate ist der flexible Einsatz auf verschiedenen Analyzern möglich und ein geringeres Volumen der Komponenten nötig.

Für Zwecke der ursprünglichen Offenbarung wird darauf hingewiesen, dass sämtliche Merkmale, wie sie sich aus der vorliegenden Beschreibung und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder chemische, physikalisch-chemische oder pharmakologische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

### Beispiele

### 1. Vorversuche

Um für das erfindungsgemäße Verfahren zur quantitativen Bestimmung der Menge an Acetaminophen eine noch höherer Präzision, eine noch höhere Sensitivität und noch weniger Wechselwirkungen und weniger spektrale und chemische Interferenzen mit anderen in der Probe enthaltenen Verbindungen erreichen zu können, wurden verschiedene Verbindungen der allgemeinen Formel (III) als Kopplungsmittel für die Umsetzung mit dem aus der Hydrolyse von Acetaminophen entstandenen p-Aminophenol untersucht.

Hierfür wurde eine Probe verwendet, deren Konzentration bei 100 mg/L Acetaminophen lag, und nach der hydrolytischen Spaltung durch Arylacylamidase erfolgte die Umsetzung mit den in der nachfolgenden Tabelle angegebenen Verbindungen Nr. 1 bis 7.

| Nr. | Name | Typ | R1 | R2 | R3 | R4 | Ext. (λ=700nm) |
|---|---|---|---|---|---|---|---|
| 1 | ALPS | Chinolin | H | H | C₂H₅ | C₃H₅SO₃Na | 0,08 |
| 2 | ADPS | Chinolin | OCH₃ | H | C₂H₅ | C₃H₆SO₃Na | 0,59 |
| 3 | TOOS | Chinolin | CH₃ | H | C₂H₅ | C₃H₅(OH)SO₃Na | 0,13 |
| 4 | DAOS | Chinolin | OCH₃ | OCH₃ | C₂H₅ | C₃H₅(OH)SO₃Na | 0,27 |
| 5 | Referenz | o-Kresol | - | - | - | - | 0,01 |

Die Verbindung Nr. 1 weist nicht die erfindungsgemäß verlangte Konstitution auf, da weder R1 noch R2 -OCH3 sind und R4 auch keinen OH-Substituenten aufweist. Die Verbindungen Nr. 2 bis 4 sind im Vergleich hierzu als erfindungsgemäß zu bezeichnen.

Dabei zeichnet sich die Verbindung Nr. 2 dadurch aus, dass R1 -OCH3 ist. Die Verbindung Nr. 2 weist somit die erfindungsgemäß verlangte Konstitution auf. Auch die Verbindungen Nr. 3 und 4 weisen eine erfindungsgemäße Konstitution auf, da bei beiden Verbindungen R4 einen OH-Substituenten aufweist. Die Verbindung Nr. 4 zeichnet sich dabei insbesondere noch dadurch aus, dass sowohl R1 als auch R2 -OCH3 sind.

Die Verbindung Nr. 5 repräsentiert den Stand der Technik, bei dem als Kopplungsmittel o-Kresol (= Ortho-Kresol bzw. 2-Methylphenol) verwendet wird.

Die photometrische Auswertung der hier untersuchten Kopplungsmittel ergab, dass bei der für Zwecke der Erfindung besonders interessierenden Wellenlänge von λ=700nm die erfindungsgemäßen Verbindungen Nr. 2 bis Nr. 4 die höchsten Extinktionswerte erreichten. Zwar liegt auch das Kopplungsmittel Nr. 5 (o-Kresol) in einem guten Bereich. Insbesondere die sehr guten Extinktionswerte der erfindungsgemäßen Kopplungsmittel Nr. 2 und Nr. 4 werden jedoch nicht erreicht. Außerdem ist o-Kresol mit dem Nachteil behaftet, dass es toxisch und von begrenzter Stabilität ist, was dazu führen kann, dass die Messzuverlässigkeit in manchen Fällen nicht über den gesamten diagnostisch relevanten Bereich mit hinreichender Zuverlässigkeit reproduzierbar ist.

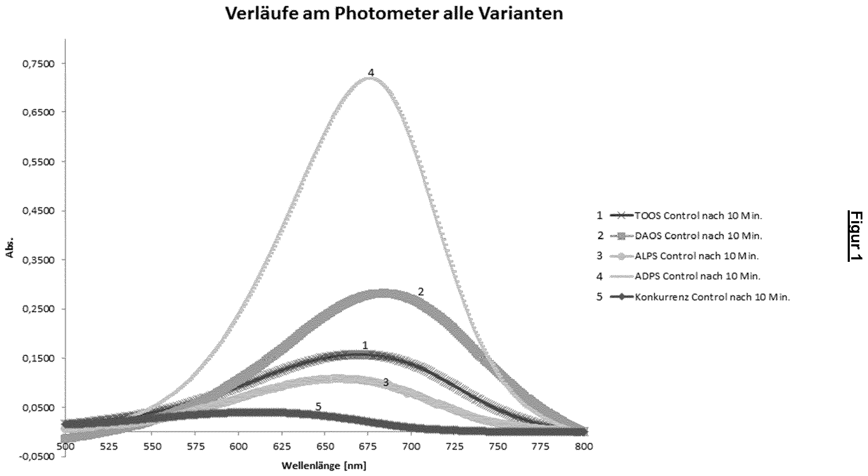

Die vollständigen Kurvenverläufe der photometrischen Auswertung der verschiedenen Kopplungsmittel über den Wellenlängenbereich von 500 nm bis 800 nm sind in der anhängenden Figur 1 dargestellt.

### 2. Ausführungsbeispiel

Bei der hier beschriebenen Ausführungsform werden die verschiedenen für die Reaktion eingesetzten Reagenzien in Form eines Reagenzien-Kits mit den folgenden Lösungen, die in separaten Gebinden vorgehalten werden, bereitgestellt. Dabei bezeichnet R1 eine erste Reagenzienlösung und R2 eine zweite Reagenzienlösung.

R1 enthält in wässriger Lösung
- Periodat (1,88 mmol/L)
- N-Ethylmaleinimid (10 mM) und weist phosphatgepuffert einen pH von 10,65 auf. R2 enthält in wässriger Lösung
- Arylacylamidase (7 U/ml)
- ADPS (10 mmol/L)
und weist phosphatgepuffert einen pH von 7,00 auf.

### 3. Versuchsbeispiel

Das Reagenzien-Kit gemäß dem unter Ziffer 2. beschriebenen Reagenzienkit wird in einem Verfahren zur Acetaminophen-Bestimmung in der folgenden Weise eingesetzt.

Vollblutproben mit verschiedenen Acetaminophen-Konzentrationen werden hämolysiert und danach mit den Reagenzienlösungen gemäß Ausführungsbeispiel 2 versetzt.

Anschließend erfolgt eine photometrische Bestimmung des mit dem Kopplungsmittel quantitativ umgesetzten p-Aminophenols.

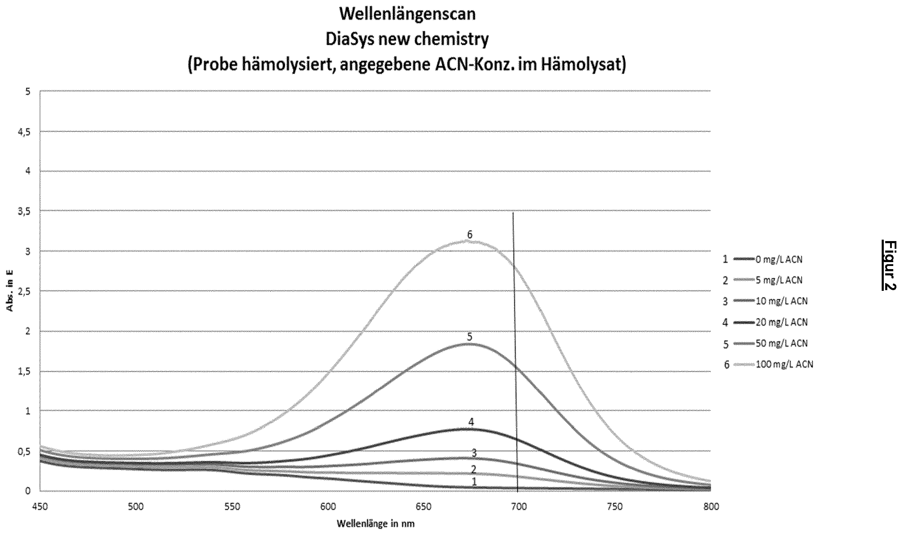

In Figur 2 sind die Ergebnisse der photometrischen Bestimmung in Form eines Wellenlängenscans dargestellt. Hier ist ersichtlich, dass bei einer Wellenlänge von 700nm die Eigenabsorption des Vollbluts keinen störenden Einfluss auf die Acetaminophen-Bestimmung nehmen kann.

## Patentansprüche

1. Verfahren zur quantitativen Bestimmung der Menge an Acetaminophen in einer Probe, **dadurch gekennzeichnet, dass** man ein Reaktionsgemisch erzeugt, indem man Acetaminophen mit der Formel (I) 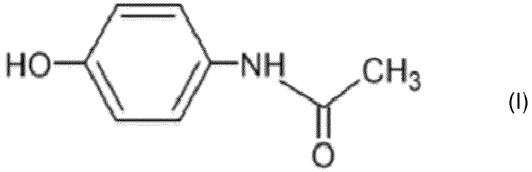 hydrolysiert und das daraus hervorgehende p-Aminophenol mit der Formel (II) 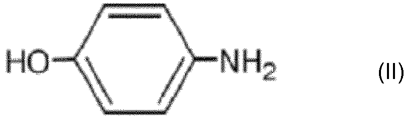 mit einer Verbindung der allgemeinen Formel (III) 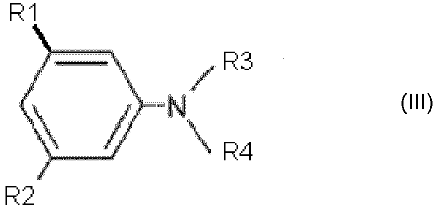 in Gegenwart eines Oxidationsmittels zu einer Verbindung der allgemeinen Formel (IV) umsetzt 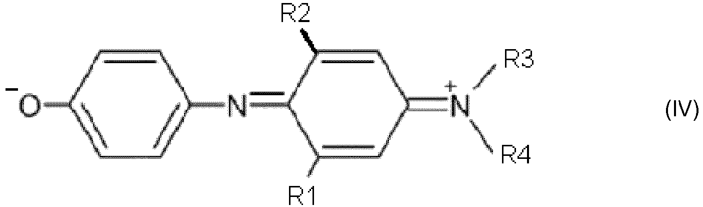 wobei in den Formeln (III) und (IV)
- R1 und R2 unabhängig voneinander ausgewählt sind unter H, -CH₃, und -OCH₃,
- R3 -C₂H₅ ist und
- R4 ein substituierter C₁₋₄-Alkylrest mit einer endständigen Sulfonatgruppe ist, mit der Maßgabe, dass wenigstens einer von R1 und R2 -OCH₃ ist und/oder R4 zusätzlich wenigstens einen OH-Substituenten aufweist,
und anschließend die Menge der Verbindung der allgemeinen Formel (IV) in dem Reaktionsgemisch photometrisch bestimmt.

2. Verfahren nach Anspruch 1, **dadurch gekennzeichnet, dass** das Oxidationsmittel unter Wasserstoffperoxid, einem organischen Peroxid, einem Eisencyanidkomplex und einem Periodatsalz oder Periodatkomplex ausgewählt ist.

3. Verfahren nach einem der Ansprüche 1 und 2, **dadurch gekennzeichnet, dass** das Absorptionsmaximum der Verbindung der allgemeinen Formel (IV) in dem Wellenlängenbereich von 650 nm bis 800 nm liegt.

4. Verfahren nach einem der Ansprüche 1 bis 3 **dadurch gekennzeichnet, dass** es in Gegenwart von N-Ethylmaleinimid und/oder Maleinimid durchgeführt wird.

5. Verfahren nach einem der Ansprüche 1 bis 4, **dadurch gekennzeichnet, dass** es in Gegenwart von EDTA durchgeführt wird.

6. Verfahren nach einem der Ansprüche 1 bis 5, **dadurch gekennzeichnet, dass** die Probe eine flüssige Probe ist und ein Volumen von 5 µL oder weniger aufweist.

7. Verfahren nach einem der Ansprüche 1 bis 6, **dadurch gekennzeichnet, dass** es in Gegenwart eines Phosphatpuffers durchgeführt wird.

8. Verfahren nach einem der Ansprüche 1 bis 7, **dadurch gekennzeichnet, dass** es in Gegenwart von Borsäure durchgeführt wird.

9. Verfahren nach einem der Ansprüche 1 bis 8, **dadurch gekennzeichnet, dass** es bei einem pH-Wert im Bereich 8 bis 10,5 durchgeführt wird.

10. Verfahren nach einem der Ansprüche 1 bis 9, **dadurch gekennzeichnet, dass** die Probe eine flüssige Probe ist und ausgewählt ist unter Blut, Blutserum oder Blutplasma.

11. Reagenziensatz zur Verwendung in einem Verfahren zur quantitativen Bestimmung von Acetaminophen in einer Probe gemäß einem der Ansprüche 1 bis 10, **dadurch gekennzeichnet, dass** der Reagenziensatz wenigstens ein Reagenz oder eine Reagenzienmischung umfasst, das oder die Arylacylamidase und eine Verbindung der allgemeinen Formel (III) enthält, und wenigstens ein Reagenz oder eine Reagenzienmischung umfasst, das oder die ein Oxidationsmittel enthält.

12. Reagenziensatz nach Anspruch 11, **dadurch gekennzeichnet, dass** der Reagenziensatz ein Reagenz 1 und ein Reagenz 2 umfasst, wobei
a) das Reagenz 1 in wässriger Lösung
- Periodat und
- N-Ethylmaleinimid enthält, und
b) das Reagenz 2 in wässriger Lösung
- Arylacylamidase und
- eine Verbindung der allgemeinen Formel (III) enthält.

13. Reagenziensatz nach Anspruch 12, **dadurch gekennzeichnet, dass** das Reagenz 1 einen pH im Bereich von 10 bis 11 aufweist.

14. Reagenziensatz nach einem der Ansprüche 12 und 13, **dadurch gekennzeichnet, dass** das Reagenz 2 einen pH im Bereich von 6 bis 8 aufweist.

15. Reagenziensatz nach einem der Ansprüche 12 bis 14, **dadurch gekennzeichnet, dass** das Volumenverhältnis der Reagenzien im Bereich von 1 Teil Reagenz 1 zu 1 Teil Reagenz 2 bis 1 Teil Reagenz 1 zu 4 Teile Reagenz 2 liegt.

## Claims

1. Method for quantitatively determining the amount of acetaminophen in a sample, **characterized in that** a reaction mixture is produced by hydrolyzing acetaminophen with formula (I) 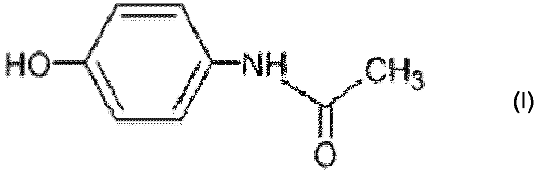 and reacting the resulting p-aminophenol with formula (II) 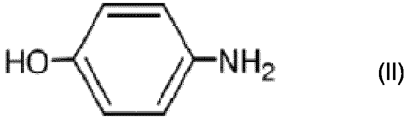 with a compound of general formula (III) 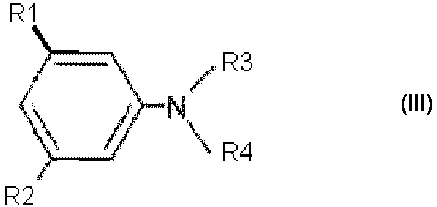 in the presence of an oxidant to form a compound of general formula (IV) 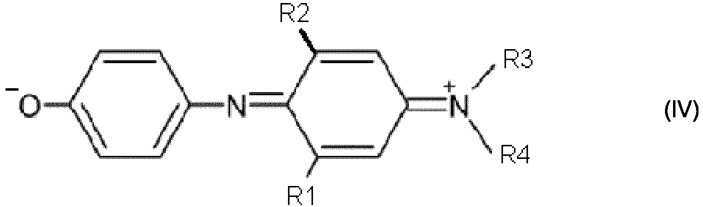 wherein, in formulae (III) and (IV),
- R1 and R2, independently of one another, are selected from H, -CH₃, and -OCH₃,
- R3 is -C₂H₅ and
- R4 is a substituted C₁₋₄ alkyl moiety with a terminal sulfonate group, with the proviso that at least one of R1 and R2 is -OCH₃ and/or R4 additionally has at least one OH substituent,
and then the amount of the compound of general formula (IV) in the reaction mixture is photometrically determined.

2. Method according to claim 1, **characterized in that** the oxidant is selected from hydrogen peroxide, an organic peroxide, an iron-cyanide complex and a periodate salt or periodate complex.

3. Method according to one of claims 1 and 2, **characterized in that** the absorption maximum of the compound of general formula (IV) lies in the wavelength range of from 650 nm to 800 nm.

4. Method according to one of claims 1 to 3, **characterized in that** it is carried out in the presence of N-ethylmaleimide and/or maleimide.

5. Method according to one of claims 1 to 4, **characterized in that** it is carried out in the presence of EDTA.

6. Method according to one of claims 1 to 5, **characterized in that** the sample is a liquid sample and has a volume of 5 µL or less.

7. Method according to one of claims 1 to 6, **characterized in that** it is carried out in the presence of a phosphate buffer.

8. Method according to one of claims 1 to 7, **characterized in that** it is carried out in the presence of boric acid.

9. Method according to one of claims 1 to 8, **characterized in that** it is carried out at a pH in the range 8 to 10.5.

10. Method according to one of claims 1 to 9, **characterized in that** the sample is a liquid sample and is selected from blood, blood serum or blood plasma.

11. Reagent set for use in a method for determining the quantity of acetaminophen in a sample according to one of claims 1 to 10, **characterized in that** the reagent set comprises at least one reagent or a reagent mixture which contains aryl acylamidase and a compound of general formula (III), and comprises at least one reagent or a reagent mixture which contains an oxidant.

12. Reagent set according to claim 11, **characterized in that** the reagent set comprises a reagent 1 and a reagent 2, wherein
a) reagent 1 contains, in aqueous solution,
- periodate and
- N-ethylmaleimide, and
b) reagent 2 contains, in aqueous solution,
- aryl acylamidase and
- a compound of general formula (III).

13. Reagent set according to claim 12, **characterized in that** reagent 1 has a pH in the range of from 10 to 11.

14. Reagent set according to one of claims 12 and 13, **characterized in that** reagent 2 has a pH in the range of from 6 to 8.

15. Reagent set according to one of claims 12 to 14, **characterized in that** the volume ratio of the reagents lies in the range of from 1 part reagent 1 to 1 part reagent 2 to 1 part reagent 1 to 4 parts reagent 2.

## Revendications

1. Procédé pour la détermination quantitative de la quantité d'acétaminophène dans un échantillon, **caractérisé en ce qu'**on produit un mélange réactionnel en hydrolysant de l'acétaminophène de formule (I), 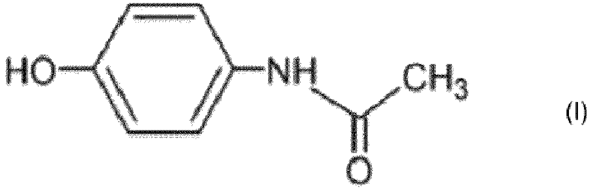 et **en ce qu'**on fait réagir le *p*-aminophénol ainsi obtenu de formule (II) 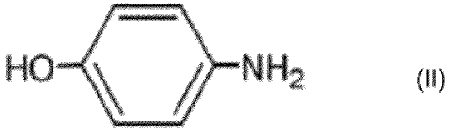 avec un composé de formule générale (III) 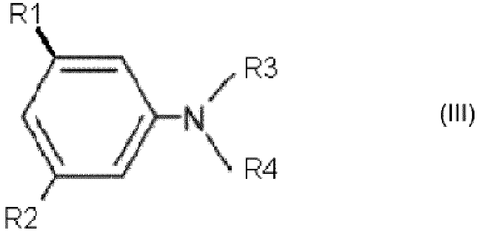 en présence d'un oxydant pour obtenir un composé de formule générale (IV) 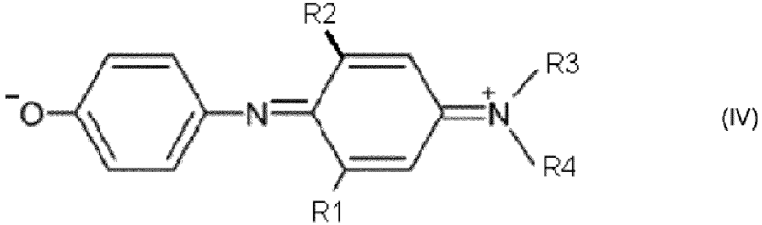 où, dans les formules (III) et (IV)
- R1 et R2 sont indépendamment l'un de l'autre choisis parmi H, -CH₃ et -OCH₃,
- R3 représente -C₂H₅, et
- R4 représente un radical alkyle en C₁₋₄ substitué ayant un groupe sulfonate terminal,
à la condition qu'au moins l'un de R1 et de R2 représente -OCH₃ et/ou que R4 présente en outre au moins un substituant OH,
la quantité du composé de formule générale (IV) dans le mélange réactionnel étant ensuite déterminée par voie photométrique.

2. Procédé selon la revendication 1, **caractérisé en ce que** l'oxydant est choisi parmi le peroxyde d'hydrogène, un peroxyde organique, un complexe de cyanure de fer et un sel periodate ou un complexe de periodate.

3. Procédé selon l'une des revendications 1 et 2, **caractérisé en ce que** le maximum d'absorption du composé de formule générale (IV) se trouve dans la plage de longueurs d'onde de 650 nm à 800 nm.

4. Procédé selon l'une des revendications 1 à 3, **caractérisé en ce qu'**il est mis en œuvre en présence de N-éthylmaléimide et/ou de maléimide.

5. Procédé selon l'une des revendications 1 à 4, **caractérisé en ce qu'**il est mis en œuvre en présence d'EDTA.

6. Procédé selon l'une des revendications 1 à 5, **caractérisé en ce que** l'échantillon est un échantillon fluide et présente un volume de 5 µL ou moins.

7. Procédé selon l'une des revendications 1 à 6, **caractérisé en ce qu'**il est mis en œuvre en présence d'un tampon phosphate.

8. Procédé selon l'une des revendications 1 à 7, **caractérisé en ce qu'**il est mis en œuvre en présence d'acide borique.

9. Procédé selon l'une des revendications 1 à 8, **caractérisé en ce qu'**il est mis en œuvre à un pH dans la plage de 8 à 10,5.

10. Procédé selon l'une des revendications 1 à 9, **caractérisé en ce que** l'échantillon est un échantillon fluide et qu'il est choisi parmi le sang, le sérum sanguin ou le plasma sanguin.

11. Kit de réactifs pour une utilisation dans un procédé de détermination quantitative d'acétaminophène dans un échantillon selon l'une des revendications 1 à 10, **caractérisé en ce que** le kit de réactifs comprend au moins un réactif ou un mélange de réactifs qui contient de l'arylacylamidase et un composé de formule générale (III), et comprend au moins un réactif ou un mélange de réactifs qui contient un oxydant.

12. Kit de réactifs selon la revendication 11, **caractérisé en ce que** le kit de réactifs comprend un réactif 1 et un réactif 2, dans lequel
a) le réactif 1 contient en solution aqueuse
- un periodate et
- du N-éthylmaléimide,
et
b) le réactif 2 contient en solution aqueuse
- de l'arylacylamidase et
- un composé de formule générale (III).

13. Kit de réactifs selon la revendication 12, **caractérisé en ce que** le réactif 1 présente un pH dans la plage de 10 à 11.

14. Kit de réactifs selon l'une des revendications 12 et 13, **caractérisé en ce que** le réactif 2 présente un pH dans la plage de 6 à 8.

15. Kit de réactifs selon l'une des revendications 12 à 14, **caractérisé en ce que** le rapport en volume des réactifs est compris dans la plage de 1 partie du réactif 1 pour 1 partie du réactif 2 à 1 partie du réactif 1 pour 4 parties du réactif 2.
